# EUROPÄISCHE PATENTANMELDUNG

(11) **EP 1 974 715 A1**
(43) Veröffentlichungstag der Anmeldung: **01.10.2008**
(21) Anmeldenummer: 07006765.7
(22) Anmeldetag: 31.03.2007
(51) Int. Cl.: A61K 8/02, A61K 8/49, A61Q 5/02, A61Q 19/10, D21H 17/14

(54) **Tränklösung für Feuchttücher**

(71) Anmelder: Zschimmer & Schwarz GmbH & Co KG Chemische Fabriken, 56112 Lahnstein (DE)
(72) Erfinder: Leikauf, Ulrich, Dr., 69245 Bammental (DE)
(74) Vertreter: Polypatent

(57) **Zusammenfassung**

Die vorliegende Erfindung betrifft eine Tränklösung zur Herstellung getränkter Tücher oder sonstiger getränkter flexibler Materialien und derartige getränkte Tücher für kosmetische, körperpflegende, dermatologische und/oder reinigende Zwecke, z.B. Reinigungstücher, Gesichtsreinigungstücher, Abschminktücher, Erfrischungstücher, Babypflegetücher, feuchte Toilettenpapiere oder Brillenputztücher, wobei die vorzugsweise wässrige Tränklösung mindestens einen Aminosäureester, vorzugsweise aus der Gruppe der Pyrrolidoncarbonsäureester, als Emulgator enthält. Die Erfindung betrifft weiterhin die Verwendung solcher Aminosäureester als Emulgatoren in Tränklösungen zur Herstellung der genannten getränkten Gewebe.

## Beschreibung

### Technisches Gebiet

Die vorliegende Erfindung betrifft Tränklösungen für Feuchttücher für kosmetische, körperpflegende, dermatologische und/oder reinigende Zwecke, wobei als Emulgator in der vorzugsweise wässrigen Tränklösung mindestens ein Aminosäureester enthalten ist. Die Erfindung betrifft weiterhin die Verwendung solcher Aminosäureester als Emulgatoren bei der Herstellung solcher Feuchttücher.

### Hintergrund der Erfindung

Derartige Gewebe bzw. Tücher finden als Gegenstände des täglichen Bedarfs Einsatz in den unterschiedlichsten Anwendungsbereichen, z.B. als Reinigungstücher, Gesichtsreinigungstücher, Abschminktücher, Erfrischungstücher, Babypflegetücher, feuchte Toilettenpapiere, Brillenputztücher, und andere feuchte Tücher. Sie erlauben eine Reinigung oder Pflege z.B. der Haut ohne die Notwendigkeit von fließendem Wasser. Dies ist auch in der geriatrischen Pflege interessant, in der nicht immer die sonst übliche Badezimmereinrichtung verwendet werden kann. Die mit einer Lösung getränkten Feuchttücher selbst bestehen in der Regel aus Papier oder anderen natürlichen oder synthetischen Fasern, wie sie beispielsweise in der EP 1029977oder US 6,338,855 beschrieben werden.

Für Gewebe oder Tissues aus natürlichen oder synthetischen Fasern ist die Tränklösung oftmals sehr verdünnt. Für Tissuepapiere werden eher Additive mit geringerem Wassergehalt bevorzugt. Herkömmliche Imprägnier- bzw.

Tränklösungen enthalten als Emulgatoren dabei häufig Polyethylenglykole und deren Abkömmlinge. So werden beispielsweise in der WO 95/35411 Lotionen für Feuchttücher vorgeschlagen, die neben Mineralöl, Fettsäureester, Fettalkoholethoxylate und Fettalkohole enthalten. Die Mehrzahl der kosmetischen Tränklösungen werden nach der sogenannten Phaseninversionstemperatur-Methode hergestellt (PIT-Methode, siehe dazu etwa K. Shinoda, H. Kunieda, Encycl. Of Emulsion Technology, 337-367, 1983), wie beispielsweise auch vorschlagen in der WO 00/04230.

Nachteilig bei diesen Tränklösungen ist, dass sie ethoxylierte Emulgatoren, i.e. Polyethylenglykol (PEG) enthaltende Emulgatoren, enthalten. Mittlerweile werden immer häufiger PEG-freie, möglichst naturnahe Emulgatortypen gefordert. Als PEG-freie Alternativen wurden dabei z.B. in der EP 1041391 die Verwendung von Citronensäureestern von Monoglyceriden und/oder Diglyceriden gesättigter Fettsäuren, in der WO 02/056841 eine Kombination aus Polyolpoly-12-hydroxystearat und Alkylpolyglycosiden und in der EP 1700618 die Kombination aus Nichtkohlenhydrat-Polyolpartialestern und Emulgatoren auf Kohlenhydratbasis vorgeschlagen. Jedoch sind auch diese Mischungen noch nicht zufriedenstellend. Die vorgeschlagenen Citronensäureester sind feste Produkte und nicht einfach zu verarbeiten. Die Alkylpolyglycoside gelten als nachteilig wegen ihrer entfettenden Wirkung auf der Haut. Andere Emulgatoren auf Kohlenhydratbasis sind nur aufwändig herzustellen und relativ hochpreisige Produkte.

Aufgabe der vorliegenden Erfindung ist es daher, Tränklösungen für Feuchttücher zur Verfügung zu stellen, die nicht die Nachteile bekannter Tränklösungen aufweisen, die also insbesondere keine ethoxylierten Emulgatoren oder Emulgatoren mit dermatologisch bedenklichen Eigenschaften enthalten.

### Beschreibung der Erfindung

Die gestellte Aufgabe wird dadurch gelöst, dass eine Tränklösung zur Verfügung gestellt wird, enthaltend:
a) mindestens einen Aminosäureester als Emulgator,
b) mindestens eine Öl- oder Wachskomponente und/oder mindestens ein weiteres Tensid, und
c) Wasser oder eine wässrige Lösung.

Unter "Tränklösung" im Sinne der vorliegenden Erfindung werden echte Lösungen, aber auch Emulsionen aus wässriger und öliger Phase verstanden. Die Emulsionen können sowohl Öl-in-Wasser- (O/W) als auch Wasser-in-ÖI-(W/O) Emulsionen sein. Bei vielen Ausführungsformen der Erfindung liegt die "Tränklösung" tatsächlich als Emulsion vor.

Bevorzugte Aminosäureester sind die Ester der Pyrrolidoncarbonsäure (PCA). Als Pyrrolidoncarbonsäureester (PCA-Ester) werden vorteilhaft die Ester aus Pyrrolidoncarbonsäure und den Fettalkoholen mit 6-30 Kohlenstoffatomen eingesetzt (C₆-C₃₀-Alkyl-PCA-Ester). Besonders vorteilhaft sind die Ester mit 8-24 Kohlenstoffatomen (C₈-C₂₄-Alkyl-PCA-Ester). Beispiele sind die Ester mit verzweigten oder unverzweigten Alkoholen, wie Capronalkohol, Caprylalkohol, 2-Ethylhexylalkohol, Nonanol, Caprinalkohol, Undecylalkohol, Laurylalkohol, Tridecylalkohol, Myristylalkohol, Cetylalkohol, Palmoleylalkohol, Stearylalkohol, Isostearylalkohol, Oleylalkohol, Elaidylalkohol, Petroselinylalkohol, Linoleylalkohol, Linolenylalkohol, Arachylalkohol, Gadoleylalkohol, Behenylalkohol, Erucylalkohol, Brassidylalkohol und die Guerbetalkohole, vorzugsweise mit 12-30 Kohlenstoffatomen. Alle genannten Ester können auch als Mischungen davon verwendet werden.

Die gestellte Aufgabe wird also dadurch gelöst, dass zur Stabilisierung von Tränklösungen für Feuchttücher Ester auf Basis von Aminosäuren, insbesondere der Pyrrolidoncarbonsäure (PCA-Ester) allein oder in Abmischung mit einem oder mehreren weiteren Tensiden verwendet werden. Prinzipiell sind solche PCA-Ester bereits bekannt. Als Emulgatoren zur Stabilisierung von Tränklösungen für Feuchttücher wurden sie bisher nicht vorgeschlagen.

Überraschenderweise wurde dabei gefunden, dass sich dabei wie mit der PIT-Methode besonders feinteilige und niedrigviskose Emulsionen herstellen lassen. Solche Emulsionen sind besonders geeignet für die Verwendung als Tränklösung für Feuchttücher.

Ein besonderer Vorteil dieser als Emulgatoren verwendeten PCA-Ester ist deren bessere Hautverträglichkeit im Vergleich zu z.B. ethoxylierten Emulgatoren. Dies trifft beispielsweise für den Lauryl-PCA-Ester zu, welcher ein sehr geringes Hautirritationspotential aufweist. Pyrrolidoncarbonsäure selbst ist als Inhaltsstoff in dem hauteigenen NMF (natural moisturizing factor) enthalten und wird bereits in kosmetischen Formulierungen als Moisturizer eingesetzt.

Ein weiterer Vorteil aus Sicht des Herstellers von Tränklösungen bzw. von entsprechenden Feuchttüchern ist die Tatsache, dass viele der PCA-Ester flüssig sind, z.B. Octyl-PCA-, Decyl-PCA- und Isostearyl-PCA-Ester. Damit lassen sich die Formulierungen - im Gegensatz zur PIT-Methode - auch kostengünstig in einem Kalt-Verfahren herstellen. Eine aufwändige Formulierung und Herstellung wie es in vielen Fällen bei entsprechenden Tränklösungen bzw. Emulsionen nach dem Stand der Technik notwendig ist, etwa mit Hilfe des PIT-Verfahrens oder eines anderen Verfahrens, bei dem die Zusammensetzungen erhitzt werden müssen, läßt sich vermeiden.

Die oben beschriebenen Aminosäureester sind in den erfindungsgemäßen Tränklösungen vorzugsweise in einem Anteil von 0,05 bis 20 Gew.-%, bevorzugter in einem Anteil von 0,1 bis 15 Gew.-% und besonders bevorzugt in einem Anteil von 0,5 bis 10 Gew.-% enthalten.

Die Tränklösungen gemäß der vorliegenden Erfindung enthalten auch mindestens eine ÖI- oder Wachskomponenten und/oder mindestens ein weiteres Tensid. Die Zusammensetzung der Tränklösung richtet sich nach dem beabsichtigten Anwendungsbereich.

So kann es vorteilhaft sein, wenn Reinigungstücher, wie z.B. Brillenputztücher, frei von Ölen oder Wachsen sind, während bei Feuchttüchern, die pflegende Eigenschaften aufweisen sollen, z.B. Gesichtsreinigungstücher, Abschminktücher oder Babypflegetücher, die Anwesenheit von Öl- oder Wachskomponenten erwünscht ist. In beiden Fällen, insbesondere jedoch bei den reinigenden Tüchern, ist die Anwesenheit einer oder mehrerer weiterer Tensidkomponenten vorteilhaft.

Die Öl- oder Wachskomponenten werden vorteilhaft aus der Gruppe der verzweigten oder unverzweigten Kohlenwasserstoffe ausgewählt. Dazu zählen z.B. Mineralöle, Paraffinöl, Paraffinwachs, mikrokristalline Wachse, Ozokerit, Ceresinwachs, Squalan und Squalen.

Als natürliche Öle können gewählt werden die pflanzlichen Öle wie z. B. Kokosöl, Palmöl, Palmkernöl, Olivenöl, Sonnenblumenöl, Sojaöl, Erdnußöl, Rapsöl, Rizinusöl, Avocadoöl, Mandelöl, Shea Butter, Sesamöl, Weizenkeimöl, Reisöl, Macadamianußöl, Lanolin und Neutral Öl.

Weiterhin können vorteilhaft gewählt werden die Fettalkohole mit 14-30 Kohlenstoffatomen wie beispielsweise Myristylalkohol, Cetylalkohol, Palmoleylalkohol, Stearylalkohol, Isostearylalkohol, Oleylalkohol, Elaidylalkohol, Petroselinylalkohol, Linoleylakohol, Linolenylalkohol, Gadoleylalkohol, Behenylalkohol, Brassidylalkohol, Erucylalkohol und die Guerbetalkohole mit 12-30 Kohlenstoffatomen.

Aus der Gruppe der Esteröle die Ester aus gesättigten und/oder ungesättigten, verzweigten und/oder unverzweigten Alkancarbonsäuren einer Kettenlänge von 3-30 C-Atomen und gesättigten und/oder ungesättigten, verzweigten und/oder unverzweigten Alkoholen einer Kettenlänge von 3-30 C-Atomen, aus der Gruppe der aromatischen Carbonsäuren und gesättigten und/oder ungesättigten, verzweigten und/oder unverzweigten Alkoholen einer Kettenlänge von 3-30 C-Atomen. Solche Esteröle können gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, 2-Ethylhexylpalmitat, 2-Ethylhexyllaurat, 2-Ethylhexylisostearat, 2-Ethylhexylcocoat, Oleyloleat, Oleylerucat, Erucylerucat, C12-15-Alkylbenzoat, aber auch Dicarbonsäureester wie die Tartrate oder Succinate von Fettalkoholen mit gleichen oder unterschiedlichen Kettenlängen, sowie synthetische und halbsynthetische und natürliche Gemische solcher Ester wie Jojobaöl und Bienenwachs, Carnaubawachs oder Candelillawachs. Zudem die Ester der Kohlensäure wie beispielsweise 2-Ethylhexylcarbonat, Dihexylcarbonat oder Dicaprylcarbonat oder die Dialkylether wie Dioctylether, Di-(2-ethylhexyl)-ether, Dicaprylether oder Didodecylether.

Vorteilhaft kann die Ölphase einen Gehalt an cyclischen oder linearen Silikonöle aufweisen oder vollständig aus solchen Ölen bestehen. Vorteilhaft wird Dimethicon oder Cyclomethicon als erfindungsgemäß zu verwendendes Silikonöl eingesetzt. Aber auch andere Silikonöle können verwendet werden, beispielsweise Hexamethylcyclotrisiloxan, Polydimethylsiloxan, Poly(methylphenylsiloxan), aber auch funktionalisierte Silikonöle.

Die erfindungsgemäßen Tränklösungen enthalten bis zu 80 Gew.-% ÖI- und/oder Wachskomponenten. Sofern vorhanden, beträgt der Anteil an ÖI- und/oder Wachskomponenten in der Tränklösung vorzugsweise 0,1 bis 70 Gew.-%. In vielen Ausführungsformen der Erfindung beträgt der Anteil besonders bevorzugt 0,5 bis 10 Gew.-%.

Als vorteilhaft hat sich auch die Kombination von Pyrrolidoncarbonsäureestern (PCA-Ester) mit weiteren Tensiden erwiesen. Unter dem Begriff "Tenside" werden grenzflächenaktive Substanzen verstanden, die an Ober- und Grenzflächen Adsorptionsschichten bilden oder in Volumenphasen zu Mizellkolloiden oder lyotropen Mesophasen aggregieren können (H.-D. Dörfler, Grenzflächen und kolloid-disperse Systeme, Springer, Berlin, Heidelberg, 2002). Es handelt sich dabei meist um niedermolekulare Verbindungen, die einen hydrophilen (polaren) Teil und einen hydrophoben (unpolaren) Rest meist langkettiger Kohlenwasserstoffe enthalten. Man unterscheidet dabei anionenaktive Tenside (Aniontenside), kationenaktive Tenside (Kationtenside), amphotere Tenside (Amphotenside) und nichtionische Tenside (Niotenside). Diesem Einteilungsprinzip liegt die Ladungsstruktur der Kopfgruppe zugrunde.

Als anionische Tenside eignen sich für die erfindungsgemäße Zubereitung, vorzugsweise in Form der Natrium-, Kalium- und Ammonium- oder der Mono-, Di- und Trialkanolammoniumsalze, sowie anderer Gegenionen, wie z.B. basische Aminosäuren, die linearen und verzweigten Fettsäuren mit 8 bis 30 Kohlenstoffatomen (Seifen), z.B. Laurinsäure, Aluminiumstearat, oder Zinkundecylenat; die Ethercarbonsäuren, wie z.B. Natriumlaureth-13-Carboxylat und Natrium-PEG-6-Cocamide-Carboxylat; Amidethercarbonsäuren; Alkylsulfate, z.B. Laurylsulfat; Fettalkoholethersulfate, z.B. Natrium-Laurethsulfat, Natriummyrethsulfat und NatriuM-C₁₂-C₁₃-Parethsulfat; Fettsäureethersulfate; Monoglycerid(ether)sulfate, z.B. Natriumcocosmonoglyceridsulfat; Alkylbenzolsulfonate; Alkansulfonate; Olefinsulfonate; Alkylethersulfonate; α-Sulfofettsäuremethylester; Sulfosuccinate, z.B. Disodium-Lauryl-Sulfosuccinat, Disodium-Laureth-Sulfosuccinat; Succinamate; Fettsäureisethionate, z.B. Natrium-Cocoyl-lsethionat; Acyllactylate, z.B. Natrium- oder Calciumstearoyllactylat; Alkyl(ether)-tartrate; Alkyl(ether)citrate, z.B. Glyceryl-Stearat-Citrat und Laureth-6-Citrat; Alkyloligoglucosidsulfate und Alkyl(ether)phosphate; sowie die Acylaminosäuren wie Acylglutamat, Caprylic/Capric-Glutamat, Palmitoylaspartat und deren Salze, aber auch von anderen natürlichen und unnatürlichen Aminosäuren; Acylpeptide beispielsweise aus Weizen-, Soja-, Reis- oder Milchprotein oder aus Kollagen; schließlich Sarcosinate wie beispielsweise Lauroyl-Sarcosin, Cocoyl-Sarcosin, Myristoyl-Sarcosin und deren Salze; und Taurate, wie beispielsweise Natriumlauroyltaurat oder Natriummethylcocoyltaurat.

Vorteilhaft zu verwendende kationische Tenside sind Alkylamine, Alkylimidazole, ethoxylierte Amine, quaternäre Tenside, insbesondere die Trialkylammoniumchloride- und bromide, wie beispielsweise Benzyldimethylstearylammoniumchlorid oder Cetyltrimethylammoniumchlorid oder -bromid, Alkyldimethylhydroxyethylammoniumchlorid oder -bromid, Dialkyldimethylammoniumchlorid oder -bromid, Dialkylethoxylatdimethylammoniumchlorid oder -bromid, Alkylpyridiniumsalze, Imidazolinderivate und Esterquats.

Zu den zu verwendenden amphoteren Tensiden gehören z.B. Alkylbetaine; Alkylamidobetaine, wie etwa Cocamidopropylbetain; Alkylamphomono- und diacetate, wie z.B. Natriumalkylamphoacetat, Dinatriumalkylamphodiacetat; Alkylamphomono- und amphodipropionate; Aminoglycinate; Sulfobetaine; Aminoxide und Phospholipide.

Als zusätzliche nichtionische Tenside können gewählt werden die Polyalkylenglykolether, -ester oder -amide. Solche Verbindungen sind beispielsweise Anlagerungsprodukte von Ethylenoxid und/oder Propylenoxid an Fettalkohole, Fettsäuren, natürliche und unnatürliche Fette und Öle, Fettsäureglyceride und Fettsäureamide. Zudem Ester, die durch Veresterung von Carbonsäuren mit Glycerin, Polyglycerin, Sorbitan, Zucker oder anderen Alkoholen entstehen, sowie deren Umsetzungsprodukte mit Alkylenoxiden oder Alkanolamide wie MEA, DEA oder MIPA-Cocamid. Außerdem Alkylpolyglycoside wie Laurylglycosid, Decylglycosid und Cocoglycosid oder Zuckertenside vom Typ der Fettsäure-N-alkylpolyhydroxylamide.

Die erfindungsgemäßen Tränklösungen enthalten bis zu 20 Gew.-% weitere Tenside. Sofern vorhanden, beträgt der Anteil an weiteren Tensiden in der Tränklösung vorzugsweise 0,5 bis 15 Gew.-% und besonders bevorzugt 1,0 bis 10 Gew.-%. In einigen Ausführungsformen der Erfindung sind die Tränklösungen frei von weiteren Tensiden.

Die Tränklösung kann vorteilhaft übliche kosmetische Hilfsstoffe enthalten, wie beispielsweise Alkohole, insbesondere solche mit niedriger C-Zahl wie Ethanol, Isopropanol, Diole oder Polyole niedriger C-Zahl, z.B. Glycerin; die Ether dieser Alkohole, wie Propylenglykol, Ethlyenglykol, Ethlyenglykolmonoethyl- oder Ethlyenglykolmonobutylether, Propylenglykolmonomethyl-, Propylenglykolmonoethyl-, Propylenglykolmonobutylether, Diethylenglykolmonomethyl-, Diethylenglykolmonoethylether, Diethylenglykolmonobutylether und analoge Produkte; Polymere; Schaumstabilisatoren; Elektrolyte; Zuckerderivate; und Moisturizer.

In bestimmten Ausführungsformen ist Glycerin ein bevorzugter Bestandteil der erfindungsgemäßen Tränklösungen bzw. der wässrigen Lösung der Tränklösungen. Es wird dann vorzugsweise in einer Menge von 0,5 bis 8 Gew.-% eingesetzt.

In bestimmten Ausführungsformen ist Ethanol ein bevorzugter Bestandteil der erfindungsgemäßen Tränklösungen bzw. der wässrigen Lösung der Tränklösungen. Es wird dann vorzugsweise in einer Menge von bis zu 15 Gew.-% eingesetzt.

Vorteilhafte Moisturizer sind beispielsweise Glycerin, Propylenglykol, Butylenglykol, Hexylenglykol, Sorbitol, Zucker, Milchsäure, Aminosäuren, Harnstoff wie beschrieben in "Skin Moisturization" Ed. James J. Leyden, Anthony V. Rawlings Marcel Dekker, 2002. Verwendbar sind auch polymere Moisturizer wie in Wasser quellbare Polysaccharide wie Hyaluronsäure, Chitosan oder auch fucosanreiche Polysacharide oder Peptide, Proteine wie z.B. Kollagen.

Die Tränklösung kann weitere Wirkstoffe, Additive und/oder Hilfsstoffe enthalten, wie beispielsweise Duftstoffe, z.B. Parfümöl; Verdickungsmittel; Konservierungsmittel; Anti-Irritationsmittel; Antioxidantien, z.B. Vitamin E; Skin Repair Actives, z.B. Ceramide, Peptide, etc.; Anti-Faltenmittel; Anti-Akne Mittel; Selbstbräunungsmittel; oder Sonnenschutzmittel.

Als Konservierungsmittel eignen sich beispielsweise Phenoxyethyanol, Parabene, Benzoesäure, Sorbinsäure, Dehydroacetsäure, Ethylhexylglycerin sowie die anderen in der europäischen Kosmetik-Richtlinie, Anlage 6, beschriebenen Stoffe, wobei die Konservierungsmittel bevorzugt als Mischungen eingesetzt werden.

Die Tränklösungen der Erfindung enthalten mindestens 10 Gew.-% Wasser oder eine wässrige Lösung, wobei unter "wässriger Lösung" eine Lösung verstanden wird, die überwiegend aus Wasser besteht.

Die Tränklösung im Sinne der vorliegenden Erfindung kann dünnflüssig sein, d.h. mit einer Viskosität von bis zu 2.000 mPas, oder in dickflüssiger Form, als Creme oder in wachsartiger Form vorliegen. In besonderen Ausführungsformen der Erfindung kann sie aber auch als oder Gel vorliegen. Vorzugsweise ist sie dünnflüssig mit einer Viskosität von bis zu 2.000 mPas.

Mit der oben beschriebenen Tränklösung können verschiedene Arten von Tüchern bzw. Geweben getränkt werden um die erfindungsgemäßen Feuchttücher zu erhalten. Diese können dann insbesondere für kosmetische, körperpflegende, dermatologische und/oder reinigende Zwecke verwendet werden.

Unter "Tuch" und unter "sonstiges flexibles Material" im Sinne der vorliegenden Erfindung werden alle Arten von gewebten oder ungewebten flexiblen Materialen verstanden, die mit den oben beschriebenen Tränklösungen getränkt werden können. Insbesondere werden darunter gewebte und ungewebte Tücher, Schwämme, watteartige Materialien und ähnliches verstanden. Als Materialien kommen alle Arten von natürlichen und/oder synthetischen Fasern in Frage. Im Falle von Schwämmen kommen natürliche oder synthetische Schwämme in Frage.

Beispiele für getränkte Tücher oder Materialien im Sinne der vorliegenden Erfindung sind Reinigungstücher, Gesichtsreinigungstücher, Abschminktücher, Erfrischungstücher, Babypflegetücher, feuchte Toilettenpapiere, Brillenputztücher, und andere feuchte Tücher.

Gegenstand der vorliegenden Erfindung ist auch ein Konzentrat zur Herstellung der oben beschriebenen Tränklösung, wobei das Konzentrat: a) mindestens einen der oben beschriebenen Aminosäureester als Emulgator, und b) mindestens eine der oben beschriebenen Öl- oder Wachskomponenten und/oder mindestens eines der oben beschriebenen weiteren Tenside enthält.

### Beispiele

### Beispiel 1: Wachsartige Tränklösung

Die Komponenten der Phase A bei 80 °C aufschmelzen und homogen rühren. Das Wasser auf 80 °C erhitzen und zur homogenen Phase A geben. Am Ultraturrax homogenisieren. Danach abkühlen. Es wird eine feste Emulsion erhalten.

| Bsp. 1 | Inhaltsstoff | Gew.-% |
|---|---|---|
| A | Olivenöl / Isopropylpalmitat | 60,0 |
| | Isostearyl-PCA | 8,0 |
| | Candelilla Wachs | 7,0 |
| B | Wasser | ad 100 |

Die wachsartige Tränklösung kann anschließend in der Wärme geschmolzen und heiß auf Papiertücher aufgesprüht werden. Die so getränkten Papiertücher können z.B. für Hygienepapierprodukte wie Taschentücher oder Toilettenpapiere verwendet werden.

### Beispiele 2 bis 7: Ölhaltige Tränklösungen

Die Komponenten der Phase A in der angegebenen Reihenfolge bei Raumtemperatur homogen mischen. Danach das Wasser bzw. Phase B unter Rühren langsam zugeben. Danach den pH auf 5,0-7,0 mit verdünnter Citronensäurelösung und/oder verdünnter Natronlauge einstellen.

| Bsp. 2 | Inhaltsstoff | Gew.-% |
|---|---|---|
| A | Paraffinum perliquidum | 5,0 |
| | Isostearyl-PCA | 2,0 |
| | Natrium Cocoyl Glutamat | 5,0 |
| | Parfümöl | 0,1 |
| | Konservierungsmittel (Phenoxyethanol, Benzoesäure, Dehydroacetsäure und Ethylhexylglycerin) | 0,5 |
| B | Glycerin | 1,0 |
| | Wasser | ad 100 |

Es wird eine feinteilige, leicht opaleszierende Emulsion erhalten.

| Bsp. 3 | Inhaltsstoff | Gew.-% |
|---|---|---|
| A | Paraffinum perliquidum | 1,5 |
| | Dodecylhexadecyl-PCA | 0,2 |
| | Isostearyl-PCA | 0,5 |
| | Natrium Cocoyl Glutamat | 1,9 |
| | Parfümöl | 0,1 |
| | Phenoxyethanol, Benzylalkohol, Kalium-Sorbat, Tocopherol | 0,5 |
| B | Dinatrium Laureth Sulfosuccinat | 2,5 |
| | Wasser | ad 100 |

Es wird eine feinteilige, fast klare Emulsion erhalten.

| Bsp. 4 | Inhaltsstoff | Gew.-% |
|---|---|---|
| A | Silikonöl 200/200 cs | 1,0 |
| | Isostearyl-PCA | 1,0 |
| | Octyl-PCA | 0,5 |
| | Cocamidopropyl Betain | 2,5 |
| | Parfümöl | 0,1 |
| | Phenoxyethanol, Methyl-, Butyl-, Ethyl-, Propyl- und Isobutylparaben | 0,5 |
| B | Wasser | ad 100 |

Es wird eine feinteilige, opaleszierende Emulsion erhalten.

| Bsp. 5 | Inhaltsstoff | Gew.-% |
|---|---|---|
| A | C12-15-Alkylbenzoat | 3,0 |
| | Decyl-PCA | 1,4 |
| | Octyl-PCA | 1,4 |
| | Natrium Cocoyl Glutamat | 7,6 |
| | Parfümöl | 0,1 |
| | Phenoxyethanol, Benzoesäure, Dehydroacetsäure, Ethylhexylglycerin | 0,75 |
| B | Cocamidopropylbetain | 7,5 |
| | Glycerin | 1,0 |
| | Wasser | ad 100 |

Es wird eine feinteilige, fast klare Emulsion erhalten.

| Bsp. 6 | Inhaltsstoff | Gew.-% |
|---|---|---|
| A | Isopropylpalmitat | 7,0 |
| | lsostearyl-PCA | 1,0 |
| | Decyl-PCA | 0,5 |
| | Natrium Cocoyl Glutamat | 3,4 |
| | Parfümöl | 0,1 |
| | Phenoxyethanol, Methyl-, Butyl-, Ethyl-, Propyl- und Isobutylparaben | 0,5 |
| B | Glycerin | 1,0 |
| | Wasser | ad 100 |

Es wird eine feinteilige, weisse Emulsion erhalten.

| Bsp. 7 | Inhaltsstoff | Gew.-% |
|---|---|---|
| A | Dicaprylether | 5,0 |
| | Octyl-PCA | 3,0 |
| | Natrium Laureth Sulfat | 4,0 |
| | Parfümöl | 0,1 |
| | Phenoxyethanol, Methyl-, Butyl-, Ethyl-, Propyl- und Isobutylparaben | 0,5 |
| B | Glycerin | 1,0 |
| | Wasser | ad 100 |

Es wird eine feinteilige, fast transparente Emulsion erhalten.

Die Tränklösungen der Beispiele 2 bis 7 können z.B. für Feuchttücher zur Händereinigung oder Hautpflege verwendet werden.

### Beispiel 8: Wässrige Tränklösung

Die Komponenten der Phase A und der Phase B separat mischen. Danach die Phase B langsam unter Rühren zur Phase A zugeben. Danach den pH auf 5,0-7,0 einstellen. Es wird eine vom Aussehen klare Lösung (micellare Lösung) erhalten.

| Bsp. 8 | Inhaltsstoff | Gew.-% |
|---|---|---|
| A | Isostearyl-PCA | 0,5 |
| | Natrium Cocoyl Glutamat | 1,4 |
| | Parfümöl | 0,1 |
| | Phenoxyethanol, Methyl-, Butyl-, Ethyl-, Propyl- und lsobutylparaben | 0,5 |
| B | Cocamidopropyl Betain | 7,5 |
| | Glycerin | 2,0 |
| | Wasser | ad 100 |

Diese Tränklösung kann z.B. für Feuchttücher zur Reinigung oder für Brillenputztücher verwendet werden.

### Beispiel 9: Alkoholische Tränklösung

Die Komponenten der Phase A in der angegebenen Reihenfolge bei Raumtemperatur homogen mischen. Danach das Wasser unter Rühren langsam zugeben. Zum Schluß unter Rühren das Ethanol zugeben. Danach den pH auf 5,0-7,0 einstellen. Es wird eine vom Aussehen opaleszierende Lösung (micellare Lösung) erhalten.

| Bsp. 9 | Inhaltsstoff | Gew.-% |
|---|---|---|
| A | lsostearyl-PCA | 1,0 |
| | Natrium Lauroyl Sarcosinat | 3,0 |
| | Parfümöl | 0,1 |
| | Phenoxyethanol, Methyl-, Butyl-, Ethyl-, Propyl- und Isobutylparaben | 0,5 |
| B | Ethanol | 8,0 |
| | Wasser | ad 100 |

Diese Tränklösung kann z.B. für Feuchttücher zur Reinigung oder für Brillenputztücher verwendet werden.

## Patentansprüche

1. Tränklösung zur Herstellung getränkter Tücher oder sonstiger getränkter flexibler Materialien für kosmetische, körperpflegende, dermatologische und/oder reinigende Zwecke enthaltend:
a) mindestens einen Aminosäureester als Emulgator,
b) mindestens eine ÖI- oder Wachskomponente und/oder mindestens ein weiteres Tensid, und
c) Wasser oder eine wässrige Lösung.

2. Tränklösung gemäß Anspruch 1, wobei der oder die Aminosäureester Ester der Pyrrolidoncarbonsäure (PCA) sind.

3. Tränklösung gemäß Anspruch 2, wobei der oder die PCA-Ester C₁-C₃₀-Alkyl-PCA-Ester, vorzugsweise C₆-C₃₀-Alkyl-PCA-Ester, besonders bevorzugt C₈-C₂₄-Alkyl-PCA-Ester, sind.

4. Tränklösung gemäß Anspruch 3, wobei der oder die Ester ausgewählt sind aus der Gruppe, die die PCA-Ester von Capronalkohol, Caprylalkohol, 2-Ethylhexylalkohol, Nonanol, Caprinalkohol, Undecylalkohol, Laurylalkohol, Tridecylalkohol, Myristylalkohol, Cetylalkohol, Palmoleylalkohol, Stearylalkohol, Isostearylalkohol, Oleylalkohol, Elaidylalkohol, Petroselinylalkohol, Linoleylalkohol, Linolenylalkohol, Arachylalkohol, Gadoleylalkohol, Behenylalkohol, Erucylalkohol, Brassidylalkohol und den Guerbetalkoholen oder Mischungen davon umfasst.

5. Tränklösung gemäß einem der vorherigen Ansprüche, wobei die mindestens eine Öl- oder Wachskomponente ausgewählt wird aus der Gruppe, die verzweigte oder unverzweigte Kohlenwasserstoffe, insbesondere Mineralöle, Paraffinöl, Paraffinwachs, mikrokristalline Wachse, Ozokerit, Ceresinwachs, Squalan und Squalen; natürliche Öle, insbesondere Kokosöl, Palmöl, Palmkernöl, Olivenöl, Sonnenblumenöl, Sojaöl, Erdnußöl, Rapsöl, Rizinusöl, Avocadoöl, Mandelöl, Shea Butter, Sesamöl, Weizenkeimöl, Reisöl, Macadamianußöl, Lanolin und Neutral-ÖI; Fettalkohole mit 14-30 Kohlenstoffatomen, insbesondere Myristylalkohol, Cetylalkohol, Palmoleylalkohol, Stearylalkohol, Isostearylalkohol, Oleylalkohol, Elaidylalkohol, Petroselinylalkohol, Linoleylakohol, Linolenylalkohol, Gadoleylalkohol, Behenylalkohol, Brassidylalkohol und Erucylalkohol; Guerbetalkohole mit 12-32 Kohlenstoffatomen; Esteröle, insbesondere Isopropylmyristat, Iso-propylpalmitat, Isopropylstearat, Isopropyloleat, n-Butylstearat, n-Hexyl-laurat, n-Decyloleat, Isooctylstearat, Isononylstearat, 2-Ethylhexylpalmitat, 2-Ethylhexyllaurat, 2-Ethylhexylisostearat, 2-Ethylhexylcocoat, Oleyloleat, Oleylerucat, Erucylerucat, C12-15-Alkylbenzoat und Tartrate oder Succinate von Fettalkoholen mit gleichen oder unterschiedlichen Kettenlängen; sowie synthetische und halbsynthetische und natürliche Gemische solcher Ester, insbesondere Jojobaöl, Bienenwachs, Carnaubawachs oder Candelillawachs; Ester der Kohlensäure, insbesondere 2-Ethylhexylcarbonat, Dihexylcarbonat oder Dicaprylcarbonat; Dialkylether, insbesondere Dioctylether, Di-(2-ethylhexyl)-ether, Dicaprylether oder Didodecylether; Silikonöle, insbesondere Dimethicon oder Cyclomethicon, Hexamethylcyclotrisiloxan, Polydimethylsiloxan und Poly(methylphenylsiloxan); oder die Mischungen der zuvor genannten Öle bzw. Wachse, umfasst.

6. Tränklösung gemäß einem der vorherigen Ansprüche, wobei die Tränklösung zusätzlich ein oder mehrere Tenside aus der Gruppe der anionischen Tenside, kationischen Tenside, amphoteren Tenside und nichtionischen Tenside oder Mischungen davon enthält.

7. Tränklösung gemäß Anspruch 6, wobei von den anionischen Tensiden eine lineare oder verzweigte Fettsäure mit 8 bis 30 Kohlenstoffatomen (Seifen); eine Ethercarbonsäure; eine Amidethercarbonsäure; ein Alkylsulfat; ein Fettalkoholethersulfat; ein Fettsäureethersulfat; ein Monoglycerid(ether)sulfat; ein Alkylbenzolsulfonat; ein Alkansulfonat; ein Olefinsulfonat; ein Alkylethersulfonat; ein α-Sulfofettsäuremethylester; ein Sulfosuccinat; ein Succinamat; ein Fettsäureisethionat; ein Acyllactylat; ein Alkyl(ether)tartrat; ein Alkyl(ether)citrat; ein Alkyloligoglucosidsulfat oder ein Alkyl(ether)phosphat; eine Acylaminosäure; ein Acylpeptid; ein Sarcosinat; ein Taurat; vorzugsweise eine Acylaminosäure, ein Acylpeptid, ein Sarcosinat, ein Taurat; ein Salz der zuvor genannten Verbindungen oder eine Mischung davon ausgewählt wird.

8. Tränklösung gemäß einem der vorherigen Ansprüche, wobei die Tränklösung oder die wässrige Lösung ein oder mehrere weitere Komponenten enthält, ausgewählt aus der Liste, die Alkohole, vorzugsweise Ethanol, Isopropanol; Diole oder Polyole, wie Glycerin; Ether, vorzugsweise Propylenglykol, Ethlyenglykol, Ethlyenglykolmonoethyl- oder Ethlyenglykolmonobutylether, Propylenglykolmonomethyl-, Propylenglykolmonoethyl-, Propylenglykolmonobutylether, Diethylenglykolmonomethyl-, Diethylenglykolmonoethylether und Diethylenglykolmonobutylether; sowie Polymere, Schaumstabilisatoren, Elektrolyte, Zuckerderivate und Moisturizer umfasst.

9. Tränklösung gemäß einem der vorherigen Ansprüche, wobei die Tränklösung oder die wässrige Lösung ein oder mehrere weitere Wirkstoffe, Additive und/oder Hilfsstoffe, ausgewählt aus der Liste, die Duftstoffe; Verdickungsmittel; Konservierungsmittel; Anti-lrritationsmittel; Antioxidantien; Skin Repair Actives; Anti-Faltenmittel; Anti-Akne Mittel; Selbstbräunungsmittel; oder Sonnenschutzmittel umfasst.

10. Tränklösung gemäß einem der vorherigen Ansprüche, wobei die Tränklösung dünnflüssig, dickflüssig, eine Creme oder ein Gel ist.

11. Getränktes Tuch oder ein sonstiges getränktes flexibles Material für kosmetische, körperpflegende, dermatologische und/oder reinigende Zwecke, wobei das Tuch oder das Material mit einer Tränklösung gemäß einem der Ansprüche 1 bis 10 getränkt ist.

12. Getränktes Tuch oder Material gemäß Anspruch 11, wobei das Tuch aus natürlichen und/oder synthetischen Fasern besteht.

13. Getränktes Tuch oder Material gemäß Anspruch 11 oder 12, wobei das getränkte Tuch oder Material ausgewählt wird aus der Gruppe, die Reinigungstücher, Gesichtsreinigungstücher, Abschminktücher, Erfrischungstücher, Babypflegetücher, feuchte Toilettenpapiere, Brillenputztücher, und andere feuchte Tücher umfasst.

14. Verwendung von Aminosäureestern, vorzugsweise Estern der Pyrrolidoncarbonsäure, als Emulgatoren in Tränklösungen zur Herstellung getränkter Tücher oder getränkter flexibler Materialien.

15. Verwendung einer Tränklösung gemäß einem der Ansprüche 1 bis 10 zur Herstellung getränkter Tücher oder getränkter flexibler Materialien.

16. Konzentrat zur Herstellung einer Tränklösung gemäß einem der Ansprüche 1 bis 10 enthaltend:
a) mindestens einen Aminosäureester als Emulgator, und
b) mindestens eine Öl- oder Wachskomponente und/oder mindestens ein weiteres Tensid.

17. Verfahren zur Herstellung einer Tränklösung gemäß einem der Ansprüche 1 bis 10, enthaltend die Schritte:
a) alle Komponenten bis auf das Wasser bzw. die Komponenten der wässrigen Lösung homogen rühren um eine Phase A zu erhalten,
b) das Wasser bzw. die wässrige Lösung zur Phase A geben,
c) homogenisieren, und danach
d) den pH-Wert auf 5,0 bis 7,0, vorzugsweise mit verdünnter Citronensäurelösung und/oder verdünnter Natronlauge, einstellen.

18. Verfahren gemäß Anspruch 17, wobei in Schritt a) die Komponenten vor und/oder während des Homogenrührens aufgeschmolzen werden, in Schritt b) das Wasser bzw. die wässrige Lösung vor der Zugabe zur Phase A bis zu einer Temperatur erwärmt wird, die der Temperatur +/- 5°C der Phase A entspricht, und, optional, nach Schritt d) die fertige Tränklösung abgekühlt wird bzw. abkühlen lässt.
